# EUROPEAN PATENT SPECIFICATION

(11) **EP 4 171 572 B1**
(45) Date of publication and mention of the grant of the patent: **21.05.2025**
(21) Application number: 21737779.5
(22) Date of filing: 23.06.2021
(51) Int. Cl.: A61K 31/5365, A61K 9/20, A61P 31/18, A61K 9/28

(54) **DISPERSIBLE TABLET FORMULATIONS COMPRISING DOLUTEGRAVIR**
DISPERGIERBARE TABLETTENFORMULIERUNGEN ENTHALTEND DOLUTEGRAVIR
FORMULATIONS DE COMPRIMÉS DISPERSIBLES COMPRENANT DOLUTÉGRAVIR

(30) Priority: 25.06.2020 GB 202009684
(43) Date of publication of application: 03.05.2023
(73) Proprietor: VIIV Healthcare Company, Wilmington, Delaware 19808 (US)
(72) Inventor: CONN, Ian Paul, Ware Hertfordshire SG12 0DP (GB); DAVIES, Mark Robert, Ware Hertfordshire SG12 0DP (GB); HEAFIELD, Joanne, Ware Hertfordshire SG12 0DP (GB); HOLTON, Michael, Ware Hertfordshire SG12 0DP (GB); MORTIMER, Neil, Ware Hertfordshire SG12 0DP (GB)
(74) Representative: Wilkinson, Johanna Elise
(86) International application number: PCT/IB2021/055533
(87) International publication number: WO 2021/260567

(56) References cited:
- WO-A1-2014/184553
- WO-A1-2015/140569
- US-A1- 2016 030 353
- US-A1- 2018 244 693
- US-A1- 2018 280 401

## Description

### FIELD OF THE INVENTION

The present invention relates to formulations comprising dolutegravir or a pharmaceutically acceptable salt thereof, processes for making such formulations, and the medical use of such formulations in the treatment of HIV infection, in particular in the treatment of HIV infection in pediatric patients.

### BACKGROUND TO THE INVENTION

Human immunodeficiency virus ("HIV") infection and related diseases are a major public health problem worldwide. Human immunodeficiency virus type 1 ("HIV-1") is a retrovirus which encodes three enzymes that are required for viral replication: reverse transcriptase, protease, and integrase. Drugs targeting reverse transcriptase, protease and integrase are in wide use and have shown effectiveness, particularly when employed in combination.

HIV infection however remains a major medical problem, with tens of millions of people still infected worldwide. The World Health Organization reported that 2.6 million children of less than 15 years of age were living with HIV-1 in 2014. Although therapeutic options for this patient population have improved, additional pediatric formulations of antiretroviral agents are needed.

Dolutegravir is an integrase strand transfer inhibitor (INSTI). Dolutegravir inhibits HIV integrase by binding to the integrase active site and blocking the strand transfer step of retroviral DNA integration which is essential for the HIV replication cycle.

The chemical name of dolutegravir is (4R,12aS)-N-[(2,4-difluorophenyl)methyl]-7-hydroxy-4-methyl-6,8-dioxo-3,4,12,12a-tetrahydro-2H-pyrido[5,6]pyrazino[2,6-b][1,3]oxazine-9-carboxamide (CAS Registry Number 1051375-16-6). Dolutegravir has the following structural formula:

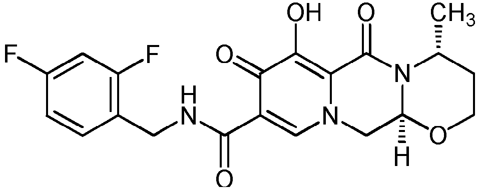

Dolutegravir sodium (TIVICAY^{®}) is approved for use in a broad population of HIV-infected patients. It is recommended for first-line treatment of HIV-1 infected adults due to it potency, high barrier to resistance and tolerability. It is also approved for children and adolescents aged 6 to 18 years of age.

The adult dosage form of TIVICAY^{®} is a tablet comprising 50mg (free acid equivalent) of dolutegravir sodium. However, some patients, in particular pediatric patients, have difficulties swallowing tablets and generally require a different oral drug delivery system. There remains a need for alternative formulations of dolutegravir or a pharmaceutically acceptable salt thereof which are suitable for use in the treatment of HIV infection in certain patients, in particular pediatric patients. WO 2015/140569 discloses a pharmaceutical composition comprising dolutegravir and one or more pharmaceutically acceptable excipients. WO 2014/184553 A1 discloses pharmaceutical antiretroviral compositions comprising a combination of antiretroviral agents. US 2016/030353 discloses a solid pharmaceutical dosage form comprising dolutegravir.

### SUMMARY OF THE INVENTION

In a first aspect, the present invention provides a dispersible tablet formulation comprising dolutegravir or a pharmaceutically acceptable salt thereof and a pharmaceutically acceptable ion, wherein the tablet comprises 5 mg of dolutegravir free acid equivalent, and wherein the dispersible tablet formulation comprises sucralose.

In a second aspect, the present invention provides a process for making a dispersible tablet formulation according to the invention, comprising dolutegravir or a pharmaceutically acceptable salt thereof and a pharmaceutically acceptable ion comprising mixing dolutegravir or a pharmaceutically acceptable salt thereof and a pharmaceutically acceptable ion.

In a third aspect, the present invention provides a dispersible tablet formulation comprising dolutegravir or a pharmaceutically acceptable salt thereof and a pharmaceutically acceptable ion, wherein the tablet comprises 5 mg of dolutegravir free acid equivalent, and wherein the dispersible tablet formulation comprises sucralose, for use in therapy.

In a fourth aspect, the present invention provides a dispersible tablet formulation comprising dolutegravir or a pharmaceutically acceptable salt thereof and a pharmaceutically acceptable ion, wherein the tablet comprises 5 mg of dolutegravir free acid equivalent, and wherein the dispersible tablet formulation comprises sucralose, for use in the treatment of HIV infection, in particular HIV infection in a pediatric patient.

Herein is also described a kit comprising a dispersible tablet formulation comprising dolutegravir or a pharmaceutically acceptable salt thereof and a pharmaceutically acceptable ion, together with instructions for the use thereof for the treatment of HIV infection.

In a further aspect, the present invention provides a combination of a dispersible tablet formulation according to the invention, comprising dolutegravir or a pharmaceutically acceptable salt thereof and a pharmaceutically acceptable ion, together with another therapeutic agent.

### DESCRIPTION OF DRAWINGS/FIGURES

Figure 1 shows the solubility of dolutegravir (in mg/ml after 30 minutes) in types of potable water.
Figure 2 is a flow diagram of the manufacturing process for preparing dolutegravir sodium granules following a high shear wet granulation process. The dolutegravir is mixed with intragranular excipients followed by the controlled addition of water to allow the nucleation, a deagglomerating wet milling process, a fluid bed drying process to dryout the granules and a dry comilling step to provide the final granule size product. The granule batch is blended with extragranular components including calcium sulfate dihydrate and a portion used to manufacture the tablet formulation.
Figure 3 shows that there is no difference in exposure in adults when 5x5mg tablets are given as a dispersion compared with 5x5mg tablets given direct to mouth.

### DETAILED DESCRIPTION OF THE INVENTION

As used herein, the term "comprise" and variations thereof, such as "comprises" or "comprising", are to be construed in an open, inclusive sense, that is as "including, but not limited to".

As used herein, the term "dispersible tablet formulation" refers to a tablet which disperses in aqueous phase, for example water, before administration or a tablet which disperses after administration direct to mouth. Typically, dispersible tablets are solid pharmaceutical forms which are defined by their rate of disintegration in water and the uniformity of dispersion of the particles into which they disintegrate. Dispersible tablet formulations may also be referred to as "tablets for oral suspension".

The formulations of the present invention may be used to treat all patients including pediatric patients, adolescent patients and adult patients. In one embodiment, the formulations of the present invention are used to treat pediatric patients. As used herein, the term "pediatric patient" refers to a child of from 0 to 12 years of age, for example 4 weeks to 6 months, 6 months to 2 years or 2 years to 6 years.

As used herein, the term "pharmaceutically acceptable" with respect to a substance refers to that substance which is generally regarded as safe and suitable for use without undue toxicity, irritation, allergic response, and the like, commensurate with a reasonable benefit/risk ratio. "Pharmaceutically acceptable" with regard to excipients includes without limitation any adjuvant, carrier, excipient, glidant, sweetening agent, diluent, preservative, dye/colorant, flavor enhancer, surfactant, wetting agent, dispersing agent, suspending agent, stabilizer, isotonic agent, solvent, or emulsifier which has been approved by the United States Food and Drug Administration as being acceptable for use in humans or domestic animals.

As used herein, the term "pharmaceutically acceptable ion" refers to an ion which is added as a solubility modifier to reduce the solubility of dolutegravir in water. Suitable ions include, but are not limited to, sodium, potassium, magnesium and calcium. In one embodiment, the pharmaceutically acceptable ion is calcium.

Thus, in one embodiment, the present invention provides the use of a pharmaceutically acceptable ion as a solubility modifier in a dispersible tablet formulation comprising dolutegravir to reduce the solubility of dolutegravir in water. Suitable ions include, but are not limited to, sodium, potassium, magnesium and calcium. In one embodiment, the pharmaceutically acceptable ion is calcium.

"Pharmaceutically acceptable salt" refers to a salt of a compound that is pharmaceutically acceptable and that possesses (or can be converted to a form that possesses) the desired pharmacological activity of the parent compound. Such salts include, but are not limited to, acid addition salts formed with inorganic acids such as hydrochloric acid, hydrobromic acid, sulfuric acid, nitric acid, phosphoric acid, and the like; or formed with organic acids such as acetic acid, benzene sulfonic acid, benzoic acid, camphorsulfonic acid, citric acid, ethane sulfonic acid, fumaric acid, glucoheptonic acid, gluconic acid, lactic acid, maleic acid, malonic acid, mandelic acid, methane sulfonic acid, 2-napththalenesulfonic acid, oleic acid, palmitic acid, propionic acid, stearic acid, succinic acid, tartaric acid, p-toluenesulfonic acid, trimethylacetic acid, and the like, and salts formed when an acidic proton present in the parent compound is replaced by either a metal ion, e.g., an alkali metal ion, an alkaline earth ion, or an aluminum ion; or coordinates with an organic base such as diethanolamine, triethanolamine, N-methylglucamine and the like. Also included in this definition are ammonium and substituted or quatemized ammonium salts. Representative non-limiting lists of pharmaceutically acceptable salts can be found in S.M. Berge et al., J. Pharma Sci., 66(1), 1-19 (1977), and Remington: The Science and Practice of Pharmacy, R. Hendrickson, ed., 21st edition, Lippincott, Williams & Wilkins, Philadelphia, PA, (2005), at p. 732, TAble 38-5.

As used herein, the term "salts" includes co-crystals. The term "co-crystal" refers to a crystalline compound comprising two or more molecular components, *e.g.* wherein proton transfer between the molecular components is partial or incomplete.

As used herein, the term "solvate" means a molecular complex comprising a compound and one or more pharmaceutically acceptable solvent molecules. Examples of solvent molecules include, but are not limited to, water and C₁₋₆ alcohols, *e.g.* ethanol. When the solvate is water, the term "hydrate" may be used.

As used herein, the term "%w/w" means the weight of a component as a percentage of the total weight of eg the granule or tablet core in which the component is present.

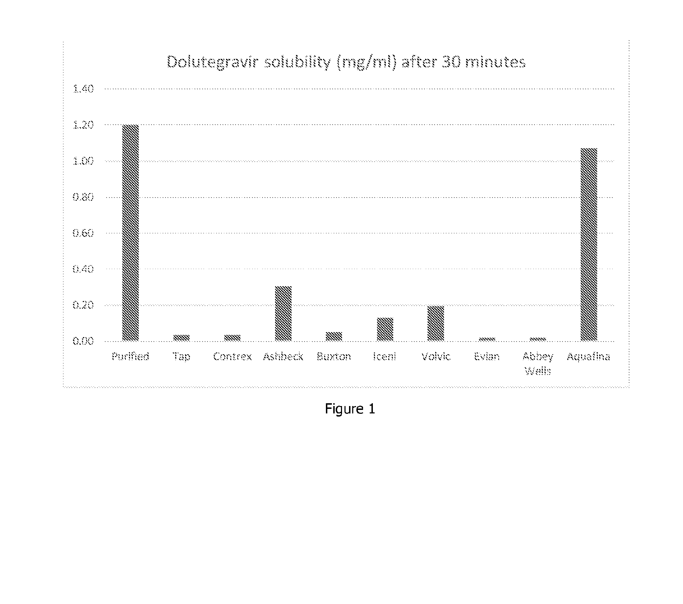

The solubility of dolutegravir is affected by the use of different types of potable water, for example purified water, tap water and different brands of bottled water (see Figure 1). The reason for this difference in solubility is believed to be due to the presence of ions typically found in water. When water with no ions present (purified water) is used to disperse dolutegravir, the solubility of dolutegravir is increased and a significant bitter taste may be observed.

The bitter taste of dolutegravir may be significantly reduced when water comprising a suitable pharmaceutically acceptable ion is used for dispersion. As the water used by patients to disperse the tablets will vary, according to the present invention a pharmaceutically acceptable ion is incorporated into a dispersible tablet formulation in order to ensure the presence of a suitable pharmaceutically acceptable ion. The dispersible tablet formulation according to the invention has improved palatability, and thus increased compliance.

In a first aspect, the present invention provides a dispersible tablet formulation comprising dolutegravir or a pharmaceutically acceptable salt thereof and a pharmaceutically acceptable ion wherein the tablet comprises 5 mg of dolutegravir free acid equivalent, and wherein the dispersible tablet formulation comprises sucralose.

In one embodiment, the dolutegravir or a pharmaceutically acceptable salt thereof is dolutegravir sodium.

According to the invention the tablet comprises 5 mg of dolutegravir, free acid equivalent.

The pharmaceutically acceptable ion is an ion which is added as a solubility modifier to reduce the solubility of dolutegravir in water. Suitable ions include, but are not limited to, sodium, potassium, magnesium and calcium. In one embodiment, the pharmaceutically acceptable ion is calcium.

The pharmaceutically acceptable ion will typically be incorporated into the dispersible tablet formulation in the form of a pharmaceutically acceptable salt, for example a sulfate salt such as calcium sulfate. The salts may be in anhydrous form or a hydate, for example a monohydrate or a dihydrate. In another embodiment, the calcium sulfate is calcium sulfate dihydrate.

In another embodiment, the pharmaceutically acceptable ion is present in an amount of up to about 5% w/w in the tablet core. In a another embodiment, the pharmaceutically acceptable ion is present in an amount of about 1 to about 4% w/w in the tablet core. In a another embodiment, the pharmaceutically acceptable ion is present in an amount of about 1.8 to about 2.6% w/w in the tablet core. In a further embodiment, the pharmaceutically acceptable ion is present in an amount of about 2.2% w/w in the tablet core.

Tablets of the invention will typically comprise one or more excipients. Excipients should be compatible with the other ingredients of the formulation and physiologically innocuous to the recipient thereof. Examples of suitable excipients are well known to the person skilled in the art of tablet formulation and may be found in, *inter alia,* "Handbook of Pharmaceutical Excipients", 7th Ed, 2012. As used herein the term "excipients" is intended to refer to, inter alia, processing aids, basifying agents, solubilisers, glidants, diluents (also known as bulking agents or fillers), binders, lubricants, surface active agents, disintegrants and the like. The term also includes agents such as sweetening agents, flavouring agents, colouring agents, preserving agents and coating agents. Such excipients will generally be present in admixture within the tablet.

Examples of processing aids include, but are not limited to, microcrystalline cellulose and silicified microcrystalline cellulose. The amount of processing aid in a tablet is generally from about 45 to about 65% w/w in the tablet core.

Examples of solubilisers include, but are not limited to, ionic surfactants (including both ionic and nonionic surfactants) such as sodium lauryl sulphate, cetyltrimethylammonium bromide, polysorbates (such as polysorbate 20 or 80), poloxamers (such as poloxamer 188 or 207), and macrogols.

Examples of lubricants, glidants and flow aids include, but are not limited to, magnesium stearate, calcium stearate, stearic acid, hydrogenated vegetable oil, glyceryl palmitostearate, glyceryl behenate, sodium stearyl fumarate, colloidal silicon dioxide, and talc. The amount of lubricant in a tablet is generally from about 0.5 to about 2% w/w in the tablet core. In one embodiment, the lubricant is sodium stearyl fumarate.

Examples of disintegrants include, but are not limited to, starches, celluloses, cross-linked PVP (crospovidone) (Type A or Type B), sodium starch glycolate (Type A or Type B), croscarmellose sodium, *etc.* In one embodiment, the disintegrant is sodium starch glycolate (Type A). In a further embodiment, the disintegrant is crospovidone (Type B). The amount of disintegrant in a tablet is generally from about 9 to about 14% w/w in the tablet core.

Examples of diluents (also known as bulking agents or fillers) include, but are not limited to, starches, maltodextrins, polyols (such as lactose), and celluloses. For example, the diluent may be selected from mannitol, microcrystalline cellulose, silicified microcrystalline cellulose and lactose monohydrate. In one embodiment, the diluent is mannitol. The amount of diluent in a tablet is generally from about 13 to about 19 % w/w in the tablet core.

Examples of binders include, but are not limited to, cross-linked PVP, HPMC, sucrose, starches, *etc.*

In one embodiment, the binder is a povidone. In a further embodiment, the binder is povidone K29/32. The amount of binder in a tablet is generally from about 1 to about 2% w/w in the tablet core.

The composition of the invention comprises sucralose. Further examples of sweetening agents include, but are not limited to, sucrose, saccharin and polyols (such as mannitol and sorbitol).

In another embodiment, the sweetening agent is present in an amount of up to about 2% w/w in the tablet core. In another embodiment, the sweetening agent is present in an amount of about 0.1% to to about 1% w/w in the tablet core. In a further embodiment, the sweetening agent is present in an amount of about 0.6 to abut 0.8% w/w in the tablet core.

Examples of flavouring agents include, but are not limited to, strawberry, orange, banana, raspberry, peach, passion fruit, golden syrup or mixtures thereof. Flavours are readily available from commercial sources such as flavour houses, or can be developed by those skilled in the art. It will be appreciated that preferred flavours assist in masking the taste of the active ingredient(s) of the formulation. In one embodiment, the flavouring agent is strawberry. Representative strawberry flavours may comprise natural flavours, synthetic equivalents thereof, or artificial flavours or mixtures thereof. In another embodiment, the strawberry flavour is strawberry cream flavour, for example PHS-132963 available from the flavour house Givaudan.

In one embodiment, the present invention provides a dispersible tablet formulation according to the invention, comprising dolutegravir or a pharmaceutically acceptable salt thereof, a pharmaceutically acceptable ion and a flavouring agent. In another embodiment, the present invention provides a dispersible tablet formulation comprising dolutegravir or a pharmaceutically acceptable salt thereof, calcium sulfate and strawberry cream flavour.

In another embodiment, the flavouring agent is present in an amount of up to about 2% w/w in the tablet core. In another embodiment, the flavouring agent is present in an amount of about 0.1% to to about 1% w/w in the tablet core. In a further embodiment, the flavouring agent is present in an amount of about 0.2 to about 0.4% w/w in the tablet core.

In one embodiment, the present invention provides a dispersible tablet formulation comprising dolutegravir or a pharmaceutically acceptable salt thereof, a pharmaceutically acceptable ion, sucralose as a sweetening agent and a flavouring agent.

In one embodiment, tablets provided herein are uncoated. In a further embodiment, tablets provided herein are coated. Although uncoated tablets may be used, it is more usual in the clinical setting to provide a coated tablet, in which case a conventional coating may be used.

Film coatings are known in the art. They can be composed of hydrophilic polymer materials and include, but are not limited to, polysaccharide materials, such as hydroxypropyl methylcellulose (HPMC), methylcellulose, hydroxyethyl cellulose (HEC), hydroxypropyl cellulose (HPC), poly(vinylalcohol-co-ethylene glycol) and other water soluble polymers. Though in one embodiment the water soluble material included in the film coating of the embodiments disclosed herein includes a single polymer material, in certain other embodiments it is formed using a mixture of more than one polymer. In one embodiment, the coating is white.

Suitable coatings include, but are not limited to, polymeric film coatings such as those comprising polyvinyl alcohol e.g. Aquarius BP18237 White film coat or Opadry OY-S-28876 white film coat. The amount of coating is generally from about 2 to about 4% w/w of the tablet core. In one embodiment, the coating is about 3% w/w of the tablet core.

In a second aspect, the present invention provides a process for making a dispersible tablet formulation according to the invention, comprising dolutegravir or a pharmaceutically acceptable salt thereof and a pharmaceutically acceptable ion comprising mixing dolutegravir or a pharmaceutically acceptable salt thereof and a pharmaceutically acceptable ion.

In one embodiment, granules of dolutegravir or a pharmaceutically acceptable salt thereof are first separately prepared prior to blending with extragranular components including a pharmaceutically acceptable ion.

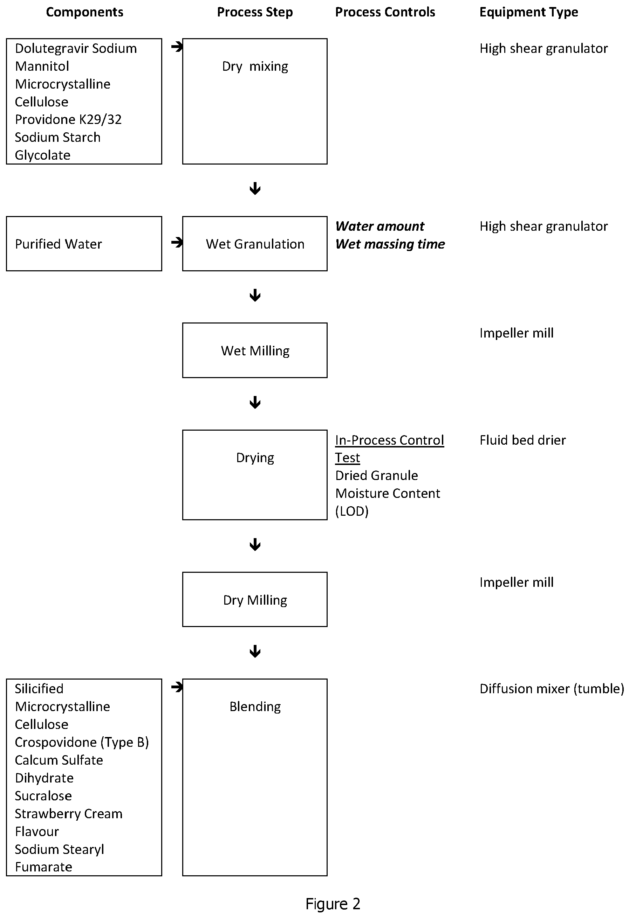

To make the dolutegravir granules, the active is first mixed with one or more of the aforementioned excipients in a suitable blender to blend the materials. In one embodiment, dolutegravir (as dolutegravir sodium) is admixed with a first amount of excipients by high shear granulation. This mixture is wet granulated and wet milled and the granules are then dried and then dry milled. Thereafter, a second amount of excipients are added to the granules and further blended. The final dolutegravir granules are collected in a suitable container. The flow diagram for dolutegravir sodium granule manufacture and subsequent blending is shown in Figure 2.

In one embodiment, the dolutegravir sodium granules comprise dolutegravir sodium, a diluent, a processing aid, a binder and a disintegrant. In another embodiment, the dolutegravir granules comprise dolutegravir sodium, mannitol, microcrystalline cellulose, povidone and sodium starch glycolate.

In one embodiment, the dolutegravir sodium granules are blended with a processing aid, a disintegrant, calcium sulfate, a sweetener, a flavouring agent and a lubricant. In another embodiment, the dolutegravir sodium granules are blended with silicified microcrystalline cellulose, crospovidone, calcium sulfate dihydrate, sucralose, strawberry cream flavour and sodium stearyl fumarate.

In one embodiment, the dispersible tablet formulation of the invention comprises 5mg free acid equivalent of dolutegravir sodium, a pharmaceutically acceptable ion in an amount of about 1.8 to about 2.6% w/w in the tablet core, a diluent in an amount of from about 13 to about 19 % w/w in the tablet core, a processing aid in an amount of about 45 to about 65% w/w in the tablet core, a binder in an amount of about 1 to about 2% w/w in the tablet core and a disintegrant in an amount of about 9 to about 14% w/w in the tablet core.

In another embodiment, the dispersible tablet formulation of the invention comprises about 5mg free acid equivalent of dolutegravir sodium, a pharmaceutically acceptable ion in an amount of about 1.8 to about 2.6% w/w in the tablet core, a sweetening agent in an amount of about 0.6 to abut 0.8% w/w in the tablet core and flavouring agent in an amount of about 0.2 to about 0.4% w/w in the tablet core.

In one embodiment, the dolutegravir sodium granules and extragranular components are blended and then compressed into a tablet.

Tabletting methods are well known in the art of pharmacy. Techniques and formulations generally are found in Remington's Pharmaceutical Sciences (Mack Publishing Co., Easton, PA). A tablet can be made by compression or moulding.

Compressed tablets may be prepared by compressing in a suitable machine the active ingredient and one or more excipients in a free-flowing form such as a powder or granules.

In another embodiment, the compressed tablet is film coated.

In one embodiment, the dispersible tablets of the invention are round with a diameter of 6 mm. In another embodiment of the invention the dispersible tablet of the invention are coated and weigh about 93mg each.

In a third aspect, the present invention provides a dispersible tablet formulation as described herein for use in therapy.

In a fourth aspect, the present invention provides a dispersible tablet formulation as described herein for use in the treatment of HIV infection, in particular HIV infection in a pediatric patient.

The dispersible tablet formulations of the invention are typically administered once per day.

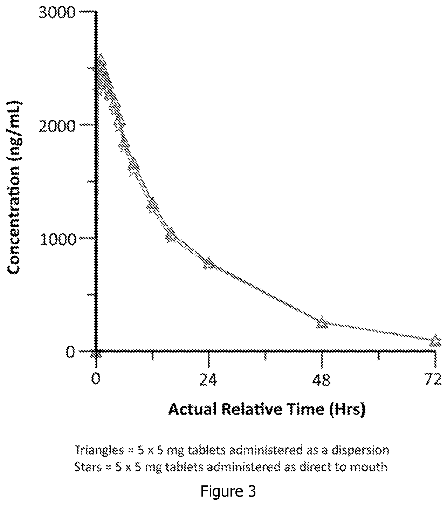

The dispersible tablet formulaitons of the invention can be dispersed in water prior to administration or administered direct to mouth (see Figure 3).

The dispersible tablet formulations of the invention are typically administered indefinitely to maintain the desired therapeutic effect. It will be further understood by such skilled artisans that suitable dosing regimens may require adjustment for an individual patient.

The dispersible tablet formulations of the invention comprise 5mg of dolutegravir in free acid form.

As the skilled person will appreciate, the number of tablets required per dose may depend on the size of the patient. In one embodiment, for children weighing from 3kg to less than 6 kg one tablet per day is dosed. In another embodiment, for children weighing from 6kg to less than 10kg 3 tablets per day are dosed. In another embodiment, for children weighing from 10kg to less than 14kg 4 tablets per day are dosed. In another embodiment, for children weighing from 14kg to less than 20kg 5 tablets per day are dosed. In a further embodiment, for children weighing 20kg and greater 6 tablets per day are dosed.

Herein is further described a kit comprising a dispersible tablet formulation according to the invention, comprising dolutegravir or a pharmaceutically acceptable salt thereof and a pharmaceutically acceptable ion, together with instructions for the use thereof for the treatment of HIV infection.

In one embodiment, the kit comprises 60 tablets, for example 60 tablets in a 60cc bottle with a child-resistant closure optionally with 2g of desiccant. In another embodiment, the kit comprises a suitable dosing system, including but not limited to a dosing cup and syringe. In a further embodiment, the kit comprises instructions regarding the amount of water in which to disperse the tablet(s). For example, for a dose of from 1 to 3 tablets, the tablets are typically dispersed in about 5ml of water. For a dose of from 4 to 6 tablets, the tablets are typically dispersed in about 10ml of water.

In a further aspect, the present invention provides a combination of a dispersible tablet formulation according to the invention, comprising dolutegravir or a pharmaceutically acceptable salt thereof and a pharmaceutically acceptable ion, together with one or more other therapeutic agents.

In one embodiment, the other therapeutic agent is a nucleoside reverse transcriptase inhibitor such as abacavir, didanosine, emtricitabine, lamivudine, stavudine, tenorfovir alafenamide fumarate, tenofovir disoproxil fumarate or zidovudine; a non-nucleoside reverse transcriptase inhibitor such as delaviridine, doravine, efavirenz, etravirine, nevirapine or rilpivirine; a protease inhibitor such as atazanavir, darunavir, fosamprenavir, indinavir, lopinavir, nelfinavir, saquinavir or tipranavir; a fusion inhibitor such as enfuvirtide; a CCR5 antagonist such as maraviroc; a cytochrome P4503A inhibitor such as ritonavir or cobicistat; an integrase inhibitor such as dolutegravir, raltegravir, elvitegravir, bictegravir or cabotegravir; or a post attachment-inhibitor such as ibalizumab-uiyk.

The individual components of such combinations may be administered either sequentially or simultaneously in separate or combined formulations.

### EXAMPLES

### Dolutegravir solubility in the presence of monovalent and divalent ions

Solubility was determined by dissolving 20mg of dolutegravir into 12 ml of water containing the specified ion (see Table 1) at a concentration of 500 ppm.

**Table 1**

| **Ion (500 ppm)** | **Dolutegravir Solubility (mg/ml)** |
|---|---|
| Sodium | 0.46 |
| Potassium | 0.78 |
| Calcium | 0.03 |
| Magnesium | 0.21 |
| None (purified water) | 1.20 |

### Dolutegravir sodium 5mg dispersible tablet

**Table 2**

| **Excipient** | **Function** | **Level (%w/w in granule)** | **Level (%w/w in tablet core) [and potential range for each level]** |
|---|---|---|---|
| **Intragranular Components** | | | |
| 5mg free acid equivalent of dolutegravir sodium | Active ingredient | | |
| Mannitol | Diluent | 50.5 | 16.2 [13.8 - 20.3] |
| Microcrystalline Cellulose | Processing Aid | 20.8 | 6.7 [5.7 - 8.4] |
| Povidone K29/32 | Binder | 5.2 | 1.7 [1.4 - 2.1] |
| Sodium Starch Glycolate (Type A) | Disintegrant | 5.2 | 1.7 [1.4 - 2.1] |
| Purified water¹ | Vehicle | q.s. | q.s. |

| **Extragranular Components** | | | |
|---|---|---|---|
| Silicified Microcrystalline Cellulose | Processing Aid | - | 53.3 [45.3 - 66.2] |
| Crospovidone (Type B) | Disintegrant | - | 10.0 [8.5 - 12.5] |
| Calcium Sulfate Dihydrate | Solubility Modifier | - | 2.2 [1.9 - 2.8] |
| Sucralose | Sweetener | - | 0.7 [0.6 - 0.8] |
| Strawberry Cream Flavour PHS-132963 | Flavour | - | 0.3 [0.3 - 0.4] |
| Sodium Stearyl Fumarate | Lubricant | - | 1.5 [1.3 - 1.9] |

| **Film Coating** | | | |
|---|---|---|---|
| Aquarious BP18237 White fim Coat or Opadry OY-S-28876 White Film Coat | Film Coat | | 2.0-4.0 [1.7 - 5] |
| Purified water | Vehicle | | q.s. |

| | | | |
|---|---|---|---|
| ¹ Purified water is removed during processing | | | |

### Specification for Dolutegravir sodium 5mg dispersible tablet

**Table 3**

| **Test** | **Acceptance Criteria** |
|---|---|
| **Description** | A white, round, biconvex tablet, with "SV H7S" debossed on one face and "5" debossed on the other face |

| **Identification of dolutegravir** | |
|---|---|
| by HPLC¹ | The retention time of the principal peak in the sample chromatogram corresponds with that of the principal peak in the dolutegravir reference material chromatogram |
| by UV¹ | The absorbance maxima in the spectrum of the sample correspond to the absorbance maxima in the reference standard |
| **Dolutegravir content by HPLC (% label claim)²** | 90.0 - 110.0 |
| **Uniformity of Dosage Units by HPLC¹** | Complies with Pharmacopoeia (USP <905>) |

| **Dolutegravir drug-related impurities content by HPLC (% area)^{3,4,5}** | |
|---|---|
| Any unspecified dolutegravir related degradation product | Not greater than 0.2 |
| Total dolutegravir degradation products | Not greater than 1.0 |
| **Fineness of dispersion⁶** | Complies with Pharmacopoeia (Ph.Eur 0478) |
| **Water content by Karl Fischer (%)⁵** | Not greater than 7.0 |
| **Dissolution by UV (% dolutegravir released)⁷** | Complies with Pharmacopoeia (USP <711>) where Q = 80% at 25 minutes |
| **Disintegration (minutes)** | ≤ 3 |
| **Microbiological Quality of Drug Product** | Less than 0.60 |
| **Water Activity (a_{w})^{1,8} (Tier 1)** | |
| **Microbial LimitsTest^{1,8} (Tier 2)** | Complies with Harmonised Pharmacopeia * |
| Total Aerobic Microbial Count (TAMC) (CFU/g) | Not greater than 10³ |
| Total combined yeasts / mould count (TYMC) (CFU/g) | Not greater than 10² |
| **Specified micro-organisms:** *Escherichia coli* | Absent in 1 g |

| | |
|---|---|
| Notes: * Ph.Eur. / USP / JP 1. Performed at release only. 2. For batch release the mean result from the uniformity of dosage units test can be applied. 3. Does not include any process/synthetic impurities. 4. All degradation products greater than or equal to 0.05% are reported. 5. Not tested at release. Performed only on stability. 6. Fineness of dispersion test will be performed on a minimum of two batches per year if manufactured. 7. Quantification of amount dissolved is determined by HPLC or UV (dissolution test). 8. If the Water Activity criterion is met then MLT (Tier 2) testing is not required. If Water Activity testing is not performed or the acceptance criterion for Water Activity is not achieved, then MLT (Tier 2) testing must be performed | |

### Taste assessment of Dolutegravir sodium 5mg dispersible tablet

The palatability and acceptability of the dispersible tablet formulation was assessed by providing respondents with the tablet and asking them to rate the taste on a scale of "very good" to "very bad". The overall assessment is shown in Table 4. The dispersible tablet formulation taste was acceptable to the majority of respondents and there were very few problems with preparation or administration.

**Table 4**

| | | |
|---|---|---|
| Number of Respondents | | 72 |

| **Overall taste assessment** | | |
|---|---|---|
| | Very Good | 15 (21%) |
| | Good | 45 (63%) |
| | Average | 10 (14%) |
| | Bad | 1 (1%) |
| | Very Bad | 1 (1%) |

## Claims

1. A dispersible tablet formulation comprising dolutegravir or a pharmaceutically acceptable salt thereof and a pharmaceutically acceptable ion, wherein the tablet comprises 5 mg of dolutegravir free acid equivalent, and wherein the dispersible tablet formulation comprises sucralose.

2. A dispersible tablet formulation according to claim 1, wherein the dolutegravir or a pharmaceutically acceptable salt thereof is dolutegravir sodium.

3. A dispersible tablet formulation according to claim 1 or claim 2 wherein the a pharmaceutically acceptable ion is calcium.

4. A dispersible tablet formulation according to claim 3 wherein the calcium is present in an amount of up to 5% w/w in the tablet core.

5. A dispersible tablet formulation according any one of the preceding claims comprising a flavouring agent.

6. A dispersible tablet formulation according to claim 5 wherein the flavouring agent is present in an amount of up to 2% w/w in the tablet core.

7. A dispersible tablet formulation according to any one of claims 1-6 wherein the sucralose is present in an amount of up to 2% w/w in the tablet core.

8. A dispersible tablet formulation according to any one of the preceding claims wherein the tablet comprises a coating.

9. A process for making a dispersible tablet formulation as claimed in any one of the preceding claims comprising mixing dolutegravir or a pharmaceutically acceptable salt thereof and a pharmaceutically acceptable ion.

10. A dispsersible tablet formulation as claimed in any one of claims 1 to 8 for use in therapy.

11. A dispsersible tablet formulation as claimed in any one of claims 1 to 8 for use in the treatment of HIV infection.

12. A combination of a dispersible tablet formulation as claimed in any one of claims 1 to 8 and another therapeutic agent selected from a nucleoside reverse transcriptase inhibitor, a non-nucleoside reverse transcriptase inhibitor, a protease inhibitor, a fusion inhibitor, a CCR5 antagonist, a cytochrome P4503A inhibitor, an integrase inhibitor, or a post attachment-inhibitor.

13. A combination as claimed in claim 12, wherein the other therapeutical agent is selected from the abacavir, didanosine, emtricitabine, lamivudine, stavudine, tenorfovir alafenamide fumarate, tenofovir disoproxil fumarate, zidovudine; delaviridine, doravine, efavirenz, etravirine, nevirapine, rilpivirine, atazanavir, darunavir, fosamprenavir, indinavir, lopinavir, nelfinavir, saquinavir, tipranavir, enfuvirtide, maraviroc, ritonavir, cobicistat, raltegravir, elvitegravir, bictegravir, cabotegravir, ibalizumab-uiyk.

## Patentansprüche

1. Dispergierbare Tablettenformulierung, umfassend Dolutegravir oder ein pharmazeutisch akzeptables Salz davon und ein pharmazeutisch akzeptables Ion, wobei die Tablette 5 mg Dolutegravir-freie-Säure-Äquivalent umfasst und wobei die dispergierbare Tablettenformulierung Sucralose umfasst.

2. Dispergierbare Tablettenformulierung gemäß Anspruch 1, wobei das Dolutegravir oder ein pharmazeutisch akzeptables Salz davon Dolutegravir-Natrium ist.

3. Dispergierbare Tablettenformulierung gemäß Anspruch 1 oder Anspruch 2, wobei das ein pharmazeutisch akzeptables Ion Calcium ist.

4. Dispergierbare Tablettenformulierung gemäß Anspruch 3, wobei das Calcium in einer Menge von bis zu 5% w/w in dem Tablettenkern vorliegt.

5. Dispergierbare Tablettenformulierung gemäß irgendeinem der vorangegangenen Ansprüche, welche einen Aromastoff (flavouring agent) umfasst.

6. Dispergierbare Tablettenformulierung gemäß Anspruch 5, wobei der Aromastoff in einer Menge von bis zu 2% w/w in dem Tablettenkern vorliegt.

7. Dispergierbare Tablettenformulierung gemäß irgendeinem der Ansprüche 1-6, wobei die Sucralose in einer Menge von bis zu 2% w/w in dem Tablettenkern vorliegt.

8. Dispergierbare Tablettenformulierung gemäß irgendeinem der vorangegangenen Ansprüche, wobei die Tablette eine Beschichtung umfasst.

9. Verfahren zum Herstellen einer dispergierbaren Tablettenformulierung wie in irgendeinem der vorangegangenen Ansprüche beansprucht, umfassend das Mischen von Dolutegravir oder eines pharmazeutisch akzeptablen Salzes davon und eines pharmazeutisch akzeptablen Ions.

10. Dispergierbare Tablettenformulierung wie in irgendeinem der Ansprüche 1 bis 8 beansprucht zur Verwendung in der Therapie.

11. Dispergierbare Tablettenformulierung wie in irgendeinem der Ansprüche 1 bis 8 beansprucht zur Verwendung bei der Behandlung von HIV-Infektion.

12. Kombination aus einer dispergierbaren Tablettenformulierung wie in irgendeinem der Ansprüche 1 bis 8 beansprucht und eines anderen therapeutischen Mittels, ausgewählt aus einem Nukleosid-reverse-Transkriptase-Inhibitor, einem Nicht-Nukleosid-reverse-Transkriptase-Inhibitor, einem Proteaseinhibitor, einem Fusionsinhibitor, einem CCR5-Antagonisten, einem Cytochrom P4503A-Inhibitor, einem Integraseinhibitor oder einem Post-Attachment-Inhibitor.

13. Kombination wie in Anspruch 12 beansprucht, wobei das andere therapeutische Mittel ausgewählt ist aus Abacavir, Didanosin, Emtricitabin, Lamivudin, Stavudin, Tenorfoviralafenamidfumarat, Tenofovirdisoproxilfumarat, Zidovudin; Delaviridin, Doravin, Efavirenz, Etravirin, Nevirapin, Rilpivirin, Atazanavir, Darunavir, Fosamprenavir, Indinavir, Lopinavir, Nelfinavir, Saquinavir, Tipranavir, Enfuvirtid, Maraviroc, Ritonavir, Cobicistat, Raltegravir, Elvitegravir, Bictegravir, Cabotegravir, Ibalizumab-uiyk.

## Revendications

1. Formulation de comprimé dispersible comprenant du dolutégravir ou un sel pharmaceutiquement acceptable de celui-ci et un ion pharmaceutiquement acceptable, dans laquelle dans laquelle le comprimé comprend 5 mg d'équivalent acide libre de dolutégravir, et dans laquelle la formulation de comprimé dispersible comprend du sucralose.

2. Formulation de comprimé dispersible selon la revendication 1, dans laquelle le dolutégravir ou un sel pharmaceutiquement acceptable de celui-ci est le dolutégravir sodique.

3. Formulation de comprimé dispersible selon la revendication 1 ou la revendication 2, dans laquelle l'ion pharmaceutiquement acceptable est le calcium.

4. Formulation de comprimé dispersible selon la revendication 3, dans laquelle le calcium est présent en une quantité allant jusqu'à 5 % p/p dans le noyau du comprimé.

5. Formulation de comprimé dispersible selon l'une quelconque des revendications précédentes, comprenant un agent aromatisant.

6. Formulation de comprimé dispersible selon la revendication 5, dans laquelle l'agent aromatisant est présent en une quantité allant jusqu'à 2 % p/p dans le noyau du comprimé.

7. Formulation de comprimé dispersible selon l'une quelconque des revendications 1 à 6, dans laquelle le sucralose est présent en une quantité allant jusqu'à 2 % p/p dans le noyau du comprimé.

8. Formulation de comprimé dispersible selon l'une quelconque des revendications précédentes, dans laquelle le comprimé comprend un enrobage.

9. Procédé de fabrication d'une formulation de comprimé dispersible selon l'une quelconque des revendications précédentes, comprenant le mélange de dolutégravir ou d'un sel pharmaceutiquement acceptable de celui-ci et d'un ion pharmaceutiquement acceptable.

10. Formulation de comprimé dispersible selon l'une quelconque des revendications 1 à 8, pour une utilisation en thérapie.

11. Formulation de comprimé dispersible selon l'une quelconque des revendications 1 à 8, pour une utilisation dans le traitement de l'infection par le VIH.

12. Association d'une formulation de comprimé dispersible selon l'une quelconque des revendications 1 à 8 et d'un autre agent thérapeutique choisi parmi un inhibiteur nucléosidique de la transcriptase inverse, un inhibiteur non nucléosidique de la transcriptase inverse, un inhibiteur de protéase, un inhibiteur de fusion, un antagoniste de CCR5, un inhibiteur du cytochrome P4503A, un inhibiteur de l'intégrase ou un inhibiteur de post-attachement.

13. Association selon la revendication 12, dans laquelle l'autre agent thérapeutique est choisi parmi l'abacavir, la didanosine, l'emtricitabine, la lamivudine, la stavudine, le fumarate de ténofovir alafénamide, le fumarate de ténofovir disoproxil, la zidovudine ; la delaviridine, la doravine, l'éfavirenz, l'étravirine, la névirapine, la rilpivirine, l'atazanavir, le darunavir, le fosamprénavir, l'indinavir, le lopinavir, le nelfinavir, le saquinavir, le tipranavir, l'enfuvirtide, le maraviroc, le ritonavir, le cobicistat, le raltégravir, l'elvitégravir, le bictégravir, le cabotégravir, l'ibalizumab-uiyk.
